# EUROPEAN PATENT APPLICATION

(11) **EP 4 609 828 A1**
(43) Date of publication of application: **03.09.2025**
(21) Application number: 23881924.7
(22) Date of filing: 26.10.2023
(51) Int. Cl.: A61F 2/24

(54) **MEDICAL DEVICE**

(30) Priority: 27.10.2022 CN 202211327871
(71) Applicant: United Innomed (Shanghai) Limited, Shanghai 201315 (CN)
(72) Inventor: WANG, Li, Shanghai 201315 (CN)
(74) Representative: Winter, Brandl - Partnerschaft mbB
(86) International application number: PCT/CN2023/126890
(87) International publication number: WO 2024/088349

(57) **Abstract**

A medical device (20) for repairing a heart valve of a patient, comprising a coaptation member (21) and a pair of blades (22) arranged at two sides of the coaptation member (21) in the thickness direction. The coaptation member (21) comprises: an extension portion (212) extending in the length direction and having one end connected to the pair of blades (22); and an acting part (211) connected to the other end of the extension part (212) and having a width greater than the width of the extension part (212). The configuration that the coaptation member (21) has an acting portion part (211) provided at one end of the coaptation member (21) and having a greater width improves the range of action of the coaptation member (21), such that only one medical device (20) is required to effectively improve the regurgitation condition of a patient with a large heart. Therefore, for a patient with a larger heart, the use of the medical device (20) can reduce the cost and difficulty of surgery.

## Description

### TECHNICAL FIELD

The present invention relates to the field of medical device technologies, and in particular, to a medical device.

### BACKGROUND

A heart valve refers to a valve located between an atrium and a ventricle or a valve located between the ventricle and an artery. The valve plays a critical role in the heart's ceaseless blood circulation. After blood flows through, the valve closes to prevent blood regurgitation. Various structural factors may affect proper closure of the heart valve, and thereby inducing the blood regurgitation. Take a mitral valve as an example, if the mitral valve fails to close properly, blood may flow from the left ventricle to the left atrium through the mitral valve during systole, and thereby impairing a patient's health.

A medical device for repairing the heart valve has a structure similar to a clip. This medical device improves the condition of valve closure insufficiency by clipping a pair of leaflets of the valve. Specifically, this device includes a coaptation member and a pair of blades located on two opposite sides in a thickness direction of the coaptation member. After this medical device is implanted into a patient's heart, one leaflet of the valve is clipped between the coaptation member and one blade, and the other leaflet of the valve is clipped between the coaptation member and the other blade. Take the mitral valve as an example, during the left ventricle diastole, the pair of the leaflets of the valve open on two sides of the medical device to form double-orifices that allow the blood flow from the left atrium to the left ventricle. During the left ventricle systole, the closure of the valve makes the double-orifices be closed to prevent the regurgitation that refers to the blood flow from the left ventricle into the left atrium.

However, because there are anatomical structure differences of the hearts of different patients, for some patients (for example, the patients with a larger heart) with more severe valvular incompetence, a single device cannot effectively mitigate the regurgitation condition of the heart. In this case, a plurality of the medical devices are required to be used, that is, a plurality of "clips" are clipped at different positions of the pair of the leaflets. This increases a cost of treatment and the difficulty of operation, and during ventricle diastole in which the valve is opened, it may also cause the valvular stenosis due to the presence of the plurality of clips is prone to causing incomplete opening, so that the blood is obstructed to flow from the atrium into the ventricle, and thus causing problem with ventricle filling occurs.

### SUMMARY

In view of this, the present invention provides a medical device for repairing a patient's heart valve to reduce a cost and difficulty of operation.

The medical device provided by the present invention includes a coaptation member and a pair of blades disposed on two sides of the coaptation member along a thickness direction of the coaptation member. The coaptation member includes an extension portion and an acting portion. The extension portion extends in a length direction, and an end of the extension portion is connected to the pair of blades. The acting portion is connected to the other end of the extension portion, and has a width greater than a width of the extension portion.

According to the medical device provided by the present invention, the coaptation member has the acting portion with a greater width, and the acting portion is provided at an end of the extension portion, this configuration expands a acting range of the coaptation member for treating incomplete closure of the valve, thereby enabling a single device to more effectively improve regurgitation in patients, and the patients includes those currently need a plurality of devices for treatment, such as patients with a relative large heart. Consequently, the medical device according to the present invention is applicable to more patients, and the cost and difficulty of operation and complications are reduced simultaneously.

In a possible implementation, the width of the acting portion is adjustable.

Since the acting portion has an adjustable width, the width may be adjusted during an operation process or after the operation according to a patient's actual heart condition, so that "precise" treatment is achieved, thereby enabling the acting portion to better match with an anatomical structure of a heart of the patient, and thus enabling the medical device to more effectively improve regurgitation.

In a possible implementation, the medical device also includes a control unit and an adjustment mechanism. The adjustment mechanism is configured to adjust the width of the acting portion under control of the control unit.

It should be considered that the patient' condition is continuously developed. When the patient's condition develops (or improves) to a certain degree, the initial width of the acting portion may no longer match with the patient's condition. According to the medical device provided by the present invention, when the patient's condition changes, the width of the acting portion is adjusted through the adjustment mechanism controlled by the control unit, so that the width of the acting portion matches with the patient's condition again. It may be seen that, if the medical device provided by the present invention is used, when the patient's condition changes, the need for adapting to the patient's condition in a manner of replacing and/or adding/replacing the medical device by re-operation or in a manner of adjusting the width of the acting portion through another interventional operation is not necessary or is delayed as much as possible. Consequently, by using the medical device provided by the present invention, the financial burden on the patient is reduced, and damage to the patient is minimized.

In a possible implementation, the medical device also includes a communication unit, the communication unit is configured to receive a control instruction, and the control unit is configured to control the adjustment mechanism to adjust the width of the acting portion based on the control instruction.

After the medical device of the present invention is implanted, the patient may perform periodic follow-up examinations to confirm the changes in the condition. When it is detected that there is a problem with the patient's transvalvular hemodynamics, for example, the problem which caused by a mismatch between the width of the acting portion and the patient's current condition, a doctor may send a control instruction from an external control device to the communication unit of the medical device implanted in the patient's body. After receiving this control instruction, the control unit may control the adjustment mechanism to appropriately adjust the width of the acting portion, and thus the width of the acting portion matches with the patient's condition again.

In a possible implementation, the medical device also includes a sensor configured to sense a physiological information of the patient, and the control unit is configured to control the adjustment mechanism to adjust the width of the acting portion based on the physiological information.

Since there is the sensor for sensing the physiological information that reflects the patient's condition, the control unit is able to adjust the width of the acting portion by controlling the adjustment mechanism based on this physiological information, so that the acting portion will better and timely match with the patient's condition. By using the medical device provided by this implementation, the number of follow-up examinations required for the patient after the implantation of the medical device is reduced, so that the economic burden and the time cost for patient is reduced, and simultaneously achieving more precise, timely, and effective treatment.

In a possible implementation, the control system also includes a wake-up unit configured to wake up one or more of the control unit, the communication unit and the sensor based on a preset schedule.

In a possible implementation, the acting portion includes a pair of shells including a first shell and a second shell. The first shell is sleeved on an outer side of the second shell, and the adjustment mechanism is configured to adjust an overlap degree of the first shell and the second shell under the control of the control unit to adjust the width of the pad.

In a possible implementation, the adjustment mechanism includes a driving unit, a pair of gears and a lead screw. The pair of gears include a first gear and a second gear meshing with each other, and the driving unit is configured to drive the first gear to rotate, such that the second gear is driven to rotate. The second gear is engaged with the lead screw, and is configured to drive the lead screw to rotate, and the lead screw is configured to drive the first shell and the second shell to move relatively to adjust the overlap degree of the first shell and the second shell.

In a possible implementation, the medical device also includes a force-applying member, and the acting portion includes a pair of shells including a first shell and a second shell, the first shell is sleeved on an outer side of the second shell, and the force-applying member is configured to adjust an overlap degree of the first shell and the second shell to adjust the width of the pad.

In a possible implementation, an outer surface of the second shell is provided with a pair of lugs, an inner surface of the first shell is provided with a pair of fixing grooves, and the pair of fixing grooves match with the pair of lugs respectively to make the acting portion has an adjustable width.

In a possible implementation, the acting portion is bent in a manner of protruding toward a side in the thickness direction. The acting portion has this configuration is capable of better matching with the shape of the pair of leaflets, so that the regurgitation is more effectively improved. In a possible implementation, a projection of the coaptation member in the thickness direction is a T-shape, and the acting portion is disposed to be perpendicular to the extension portion.

In a possible implementation, each blade includes a main body portion and a clipping seat portion. The main body portion extends in the length direction, and an end of the main body portion is connected to the coaptation member. The clipping seat portion is disposed at the other end of the main body portion, and a width of the clipping seat portion is greater than a width of the main body portion. This configuration is able to expand an acting range of the medical device, so that effectiveness of the medical device is improved, and thus the regurgitation is better improved.

In a possible implementation, the acting portion and the clipping seat portion of each blade are bent in a manner of protruding toward a same side in the thickness direction. The medical device with this configuration is able to match with the shape of the pair of leaflets, so that the regurgitation is more effectively improved.

In a possible embodiment, a ratio of the width of the acting portion to the width of the extension portion ranges from 1.25 to 5.5. Specifically, the ratio of the width of the acting portion to the width of the extension portion ranges from 2 to 4.

In a possible implementation, a ratio of the width of the clipping seat portion to the width of the main body portion ranges from 1.25 to 5.5. Specifically, the ratio of the width of the clipping seat portion to the width of the main body portion ranges from 2 to 4.

In a possible implementation, a projection the blade in the thickness direction is a T-shape, and the clipping seat portion has a structure matching with the acting portion.

### IBRIEF DESCRIPTION OF THE DRAWINGS

To more clearly illustrate the technical solutions of embodiments of the present invention, the drawings required for using in the embodiments will be briefly introduced below.

It should be understood that, the following drawings show merely some embodiments of the present invention, but not all embodiments.

It should be understood that, the same or similar reference numerals are used in the drawings to represent the same or similar elements.

It should be understood that, the drawings are merely illustrative, and sizes and proportions of elements in the drawings are not necessarily accurate.
FIG. 1A and FIG. 1B show a healthy heart valve viewed from the first chamber side, the heart valve is opened in FIG. 1A, and the heart valve is properly closed in FIG. 1B.
FIG. 2 shows a dysfunctional heart valve that fail to close properly viewed from the first chamber side.
FIG. 3 is a schematic structural diagram of a medical device according to the related art.
FIG. 4 shows the medical device shown in FIG. 3 that is mounted at a patient's heart valve.
FIG. 5A and FIG. 5B show the medical device shown in FIG. 3 that is mounted at a patient's heart valve viewed from the first chamber side, a pair of leaflets form double-orifices on two sides of the medical device in FIG. 5A, and a pair of leaflets are properly closed in FIG. 5B.
FIG. 6 shows the medical device shown in FIG. 3 that is mounted at a patient's heart valve viewed from the first chamber side, and the pair of leaflets fail to close properly.
FIG. 7 is a schematic structural diagram of a medical device according to an embodiment of the present invention.
FIG. 8 is a schematic structural diagram of the medical device shown in FIG. 7 viewed from another perspective.
FIG. 9 is a schematic structural diagram of the medical device shown in FIG. 7 viewed from yet another perspective.
FIG. 10 is a schematic structural diagram of at least a portion of a coaptation member of the medical device in FIG. 7.
FIG. 11 shows the medical device shown in FIG. 7 that is mounted at a patient's heart valve.
FIG. 12 shows the medical device shown in FIG. 7 that is mounted at a patient's heart valve viewed from the first chamber side, and a pair of leaflets are properly closed.
FIG. 13A and FIG. 13B shows a schematic structural diagram of components including an adjustment mechanism, a control system, a sensor, and an acting portion of a coaptation member of a medical device according to another embodiment of the present invention.
FIG. 14 shows an exploded schematic view of an acting portion of a coaptation member of a medical device according to another embodiment of the present invention.
FIG. 15 shows a partial cross-sectional view of a shell of the acting portion shown in FIG. 14.
FIG. 16 shows a schematic structural diagram of a medical device according to another embodiment of the present invention.
FIG. 17 shows a schematic structural diagram of the medical device shown in FIG. 16 that is viewed from another perspective.
FIG. 18 shows a schematic structural diagram of a medical device according to another embodiment of the present invention.
FIG. 19 shows a schematic structural diagram of the medical device shown in FIG. 18 that is viewed from another perspective.
FIG. 20 shows a schematic structural diagram of at least a portion of a blade of the medical device shown in FIG. 18.

### DETAILED DESCRIPTION OF THE EMBODIMENTS

Technical solutions of embodiments of the present invention will be described exemplarily with reference to the drawings below. Apparently, the described embodiments are merely a portion of implementations of the present invention, but not all the embodiments.

A heart valve refers to a valve located between an atrium and a ventricle or a valve located between the ventricle and an artery. The valve plays a critical role in the heart's ceaseless blood circulation. After blood flows through, the valve closes to prevent blood regurgitation.

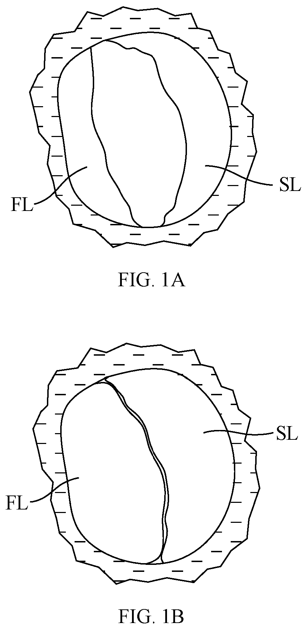

Taking a valve between a first chamber and a second chamber as an example, referring to FIG. 1A and FIG. 1B, the valve includes a pair of leaflets FL, SL. As shown in FIG. 1A, when the second chamber is in diastole, a pair of leaflets FL, SL are separated from each other to open the valve, so that blood flows from the first chamber to the second chamber. As shown in FIG. 1B, for a healthy valve, during systole of the second chamber, the pair of leaflets FL, SL properly coaptate to close the valve properly to prevent the blood flowing from the second chamber back to the first chamber.

In an example, in the present invention, the first chamber may refer to a left atrium, the second chamber may refer to a left ventricle, and the valve therebetween may refer to the mitral valve.

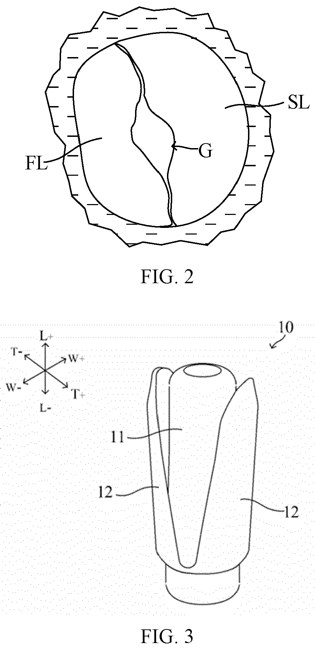

Some structural factors may affect closure of the heart valve, and thereby inducing blood regurgitation. As shown in FIG. 2, for a dysfunctional heart valve, during the systole of the second chamber, the pair of leaflets FL and SL fail to achieve proper coaptation, so that there is a gap G between them. This pathological condition will prevent the proper closure of the valve, and the blood is able to flow through the gap G from the second chamber back into the first chamber (that is, causing regurgitation), and thus impairing a patient's health.

Referring to FIG. 3, a medical device 10 includes a coaptation member 11 and a pair of blades 12. The pair of blades 12 are respectively located on two opposite sides of the coaptation member 11 in a thickness direction of the coaptation member 11. That is, a direction from one blade 12 to the other blade 12 may be regarded as the thickness direction of the medical device 10.

It should be noted that, in the drawings of the present invention, an arrow T+ and an arrow T- respectively indicate two opposite sides in the thickness direction of the medical device; an arrow L+ and an arrow L- respectively indicate two opposite sides in the length direction of the medical device; and an arrow W+ and an arrow W- respectively indicate two opposite sides in the width direction of the medical device.

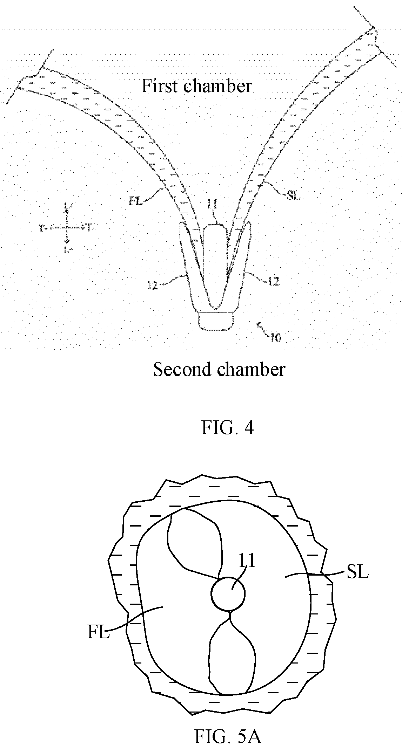

Referring to FIG. 4, after the medical device 10 is implanted at the valve site, the pair of blades 12 are respectively configured to clip the pair of leaflets FL, SL to retain the coaptation member 11 between the pair of leaflets FL, SL, so that the valve function is repaired.

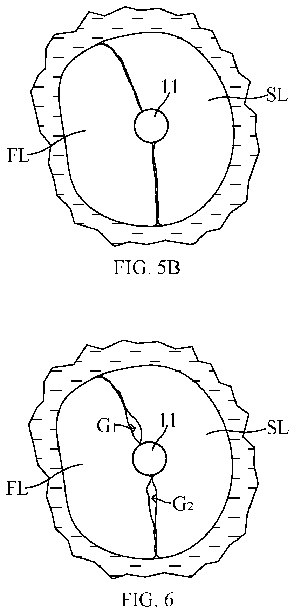

Specifically, as shown in FIG. 5A, when the second chamber is in diastole, the pair of leaflets FL, SL form double orifices on two sides of the medical device 10 (that is, on two opposite sides of the coaptation member 11). At this point, the valve is opened, so that the blood flows from the first chamber to the second chamber. As shown in FIG. 5B, during systole of the second chamber, the pair of leaflets FL, SL properly coaptate on two sides of the coaptation member 11. At this point, the valve is properly closed to prevent blood regurgitation.

However, because there are anatomical structure differences of the heart valves of hearts of different patients (for example, some patients have a larger heart), a single medical device 10 cannot effectively eliminate the regurgitation, so that there is residual regurgitation, this will still cause a high atrium pressure of the patient, so that an aortic ejection volume is reduced, and thus inducing corresponding symptoms. As shown in FIG. 6, for such patients, during the systole of the second chamber, the coaptation of the pair of leaflets FL, SL at two sides of the coaptation member 11 fails to fully eliminate the existing gap G, but instead forms two residual gaps G1 and G2 on two sides of the coaptation member 11. In this case, the blood may flow from the second chamber back to the first chamber through the gaps G1 and G2.

For such patients, it is usually necessary to implant multiple medical devices 10 either adjacently or at intervals on the heart valve. However, using the multiple devices 10 not only significantly increases a treatment cost, but also greatly increases the surgical difficulty. Furthermore, the simultaneous use of the multiple devices 10 may cause excessively restraint on the valve, so that their opening is affected during the diastole (stenosis), and thus affecting the flow of blood from the atrium into the ventricle.

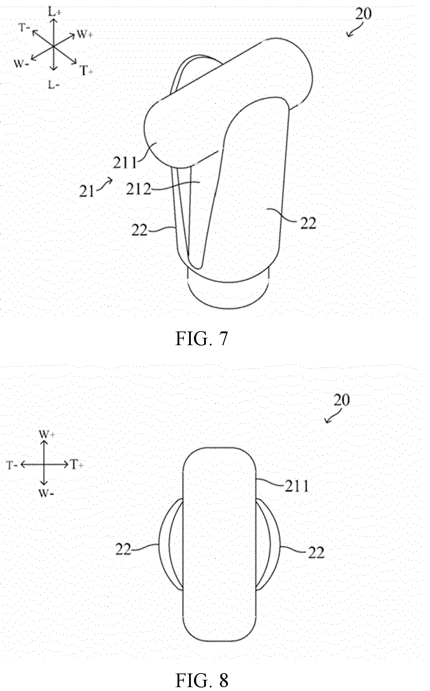

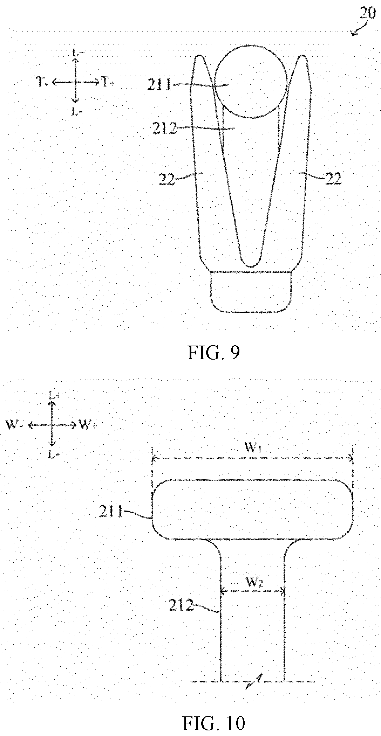

In view of the foregoing, an embodiment of the present invention provides a medical device 20. Referring to FIG. 7 to FIG. 11, the medical device 20 includes a coaptation member 21 and a pair of blades 22. The pair of blades 22 are located at two opposite sides of the coaptation member 21 in a thickness direction of the coaptation member 21. After the medical device 20 is implanted at the valve site, each blade 22 is configured to maintain the coaptation member 21 between the pair of leaflets FL, SL. In an implementation, each blade 22 may has a clip-like structure to clip the corresponding leaflet, and thereby positioning the coaptation member 21 between the pair of leaflets FL, SL. In another implementation, each blade 22 may cooperate with the coaptation member 21 to clip the corresponding leaflet therebetween, so that the coaptation member 21 is positioned between the pair of leaflets FL, SL. It should be understood that, specific implementations of the pair of blades 22 may refer to the related art, which are omitted herein for brevity.

The coaptation member 21 includes an acting portion 211 and an extension portion 212. The extension portion 212 extends in a length direction of the medical device 20, and has two opposite ends in a length direction thereof. An end of the extension portion 212 is connected (directly or via an intermediate member) to the blades 22, and the other end is connected to the acting portion 211. A width W₁ of the acting portion 211 is configured to be greater than a width W₂ of the extension portion 212.

In the present invention, the width of an element refers to its dimension in the width direction. Furthermore, in some embodiments of the present invention, the width direction, thickness direction, and length direction may be mutually perpendicular. Additionally, in some embodiments of the present invention, the width of an element may refer to its maximum width, and in other embodiments of the present invention, the width of an element may refer to its average width.

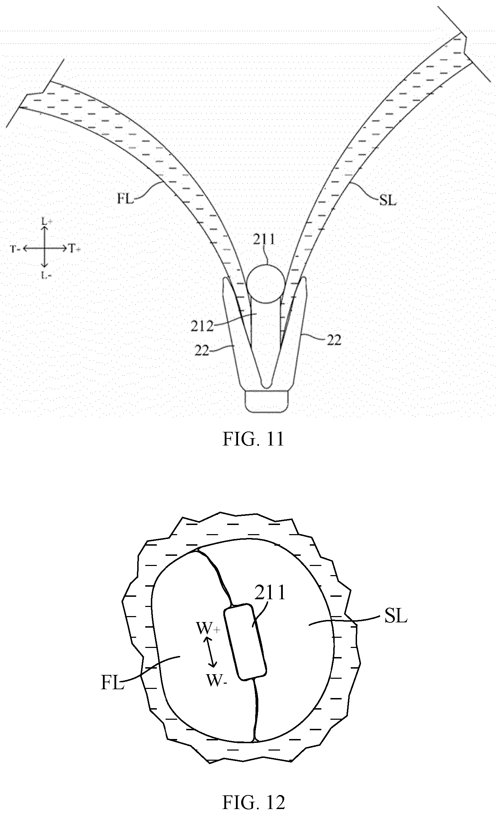

Referring to FIG. 12, after being implanted at the valve site, an extension direction of boundaries of the pair of leaflets FL, SL is substantially close to a width direction of the acting portion 211. Since the acting portion 211 has a relatively large width, so that this configuration expands the acting range of the coaptation member 21. Compared FIG. 6 and FIG. 12, it may be seen that, for a larger heart, if the medical device 20 is used, a single medical device 20 suffices to enable the pair of leaflets (FL, SL) to close properly during the systole of the second chamber, so that the regurgitation is effectively prevented or mitigated.

According to the medical device provided by the present invention, the coaptation member has an acting portion provided at an end of the extension portion with a larger width. This configuration expands the acting range of the coaptation member, such that the regurgitation in the patient with a larger heart is effectively improved by using merely a single medical device. Consequently, for the patient with a larger heart, by using the medical device provided by the present invention, the cost and difficulty of operation is reduced, and a better therapeutic effect is achieved.

It should be noted that, in the present invention, the width W1 of the acting portion 211 is not merely slightly greater than the width W2 of the extension portion 212, but rather more significantly greater than the width W2 of the extension portion 212, so that the medical device 20 is able to function as the multiple conventional medical devices in the larger heart. For example, a ratio of the width W1 of the acting portion 211 to the width W2 of the extension portion 212 ranges from 1.25 to 5.5. Specifically, the ratio of the width W₁ of the acting portion 211 to the width W₂ of the extension portion 212 ranges from 2 to 4. As shown in FIG. 7 to FIG. 10, specifically, an extension direction of the acting portion 211 is the same as the width direction, and an extension direction of the extension portion 212 is the same as the length direction. A projection of the coaptation member 21 in a thickness direction is substantially a T-shape, and the acting portion 211 is disposed to be perpendicular to the extension portion 212 and has a substantially cylindrical structure.

Considering that there are anatomical structure differences of heart valves of different patients, and the anatomical structure of the heart valve of a patient will change with disease development, and therefore, in some embodiments the coaptation member of the medical device provided by the present invention is constructed to have an adjustable width. Since the acting portion has the adjustable width, so that the width of the acting portion will be adjusted according to the actual condition of the patient's heart during or after the operation, thereby the acting portion is able to match with the anatomical structure of the patient's heart, and thus the regurgitation is more effectively improved by the medical device.

The medical device has an acting portion with an adjustable width will be illustrated with examples with reference to the drawings below.

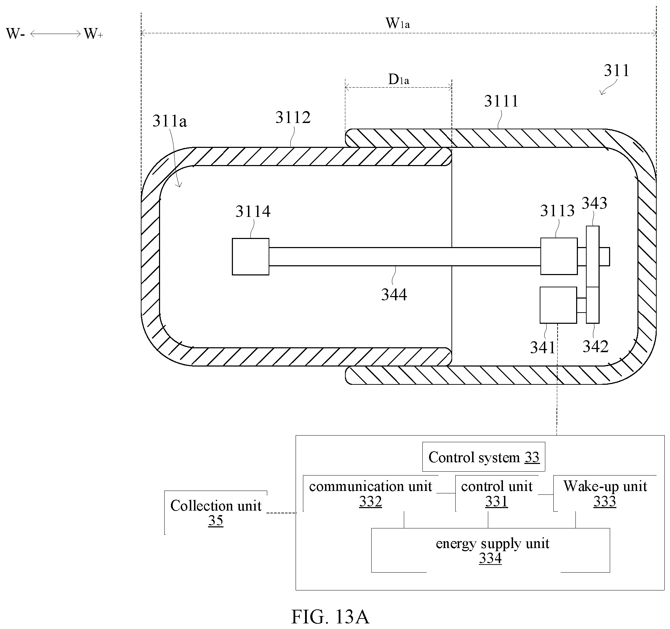

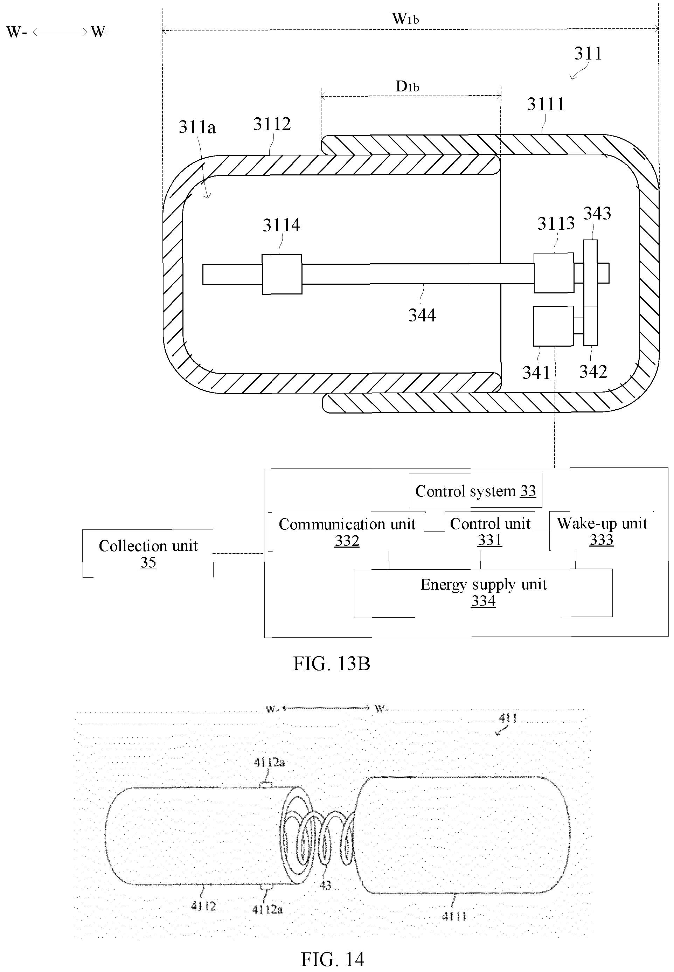

As a possible implementation, referring to FIG. 13A and FIG. 13B, a medical device according to another embodiment of the present invention may also include a control system 33 and an adjustment mechanism in addition to including the coaptation member and pair of blades. The construction of the coaptation member and blades of the medical device provided by this embodiment may refer to the aforementioned embodiments, which will not be repeated herein for the sake of brevity. The control system 33 may include a control unit 331. The adjustment mechanism may be configured to adjust the width of the acting portion 311 of the coaptation member under the control of the control unit 331.

It should be considered that the patient's condition is continuously developed. When the patient's condition develops to a certain degree, the initial width of the acting portion may no longer match with the patient's condition. According to the medical device provided by the present invention, when the patient's condition changes, the width of the acting portion is adjusted through the adjustment mechanism controlled by the control unit, so that the width of the acting portion matches with the patient's condition again. It may be seen that, if the medical device provided by the present invention is used, when the patient's condition changes, the need for adapting to the patient's condition in a manner of replacing and/or adding/replacing the medical device by re-operation or in a manner of adjusting the width of the acting portion through another interventional operation is not necessary or is delayed as much as possible. Consequently, by using the medical device provided by the present invention, the financial burden on the patient is reduced, and damage to the patient is minimized.

The adjustment of the acting portion's width may be achieved through various manners, which is not limited in the present invention. As an example, referring again to FIG. 13A and FIG. 13B, the acting portion 311 includes a pair of shells, namely a first shell 3111 and a second shell 3112. The first shell 3111 is sleeved outside the second shell 3112, and they define an internal space 311a together. In some embodiments, the first shell 3111 may be connected to the extension portion of the coaptation member.

The adjustment mechanism is configured to adjust an overlap degree of the first shell 3111 and second shell 3112 under the control of the control unit, such that the width of a pad 311 is adjusted. When the pad 311 is in a state shown in FIG. 13A, the overlap degree D₁ₐ of the first shell 3111 and the second shell 3112 is less, and the width W₁ₐ of the acting portion 311 is greater. When the acting portion 311 is in a state shown in Figure 13B, the overlap degree D_{1b} of the first shell 3111 and the second shell 3112 is greater, and the width W1b of the acting portion 311 is less.

In this way, the adjustment mechanism is able to adjust the width of the pad 311 under the control of the control unit. This implementation has many advantages, such as a relatively simple implementation, a more compact structure, and the better reliability.

There are various ways to adjust the overlap degree of the pair of shells, and the present invention does not specifically limit this.

As an example, referring again to FIG. 13A and FIG. 13B, the adjustment mechanism may include a driving unit 341, a pair of gears and a lead screw 344. An inner wall of the first shell 3111 is provided with a first boss 3113, and an inner wall of the second shell 3112 is provided with a second boss 3114, the first boss 3113 is provided with a through hole, and the second boss 3114 is provided with a threaded hole.

The pair of gears include a first gear 342 and a second gear 343, and a driving unit 341 is configured to drive the first gear 342 to rotate. For instance, the driving unit 341 may be a motor, and the first gear 342 is mounted on its output shaft. The second gear 343 is mounted on the lead screw 344 and meshes with the first gear 342. The lead screw 344 passes through the through hole of the first boss 3113 and is supported in a manner that allows it to rotate but prevents it from sliding axially via components such a bearing and a retaining ring, and the like. The lead screw 344 passes through the threaded hole of the second boss 3114 and has an external thread that matches with an external thread.

When it is necessary to adjust the width of the acting portion 311, the control unit 331 control the driving unit 341 to run. As the driving unit 341 moves, a torque is transmitted to the lead screw 344 via the first gear 342 and the second gear 343. As the lead screw 344 rotates, it makes the first shell 3111 and second shell 3112 move relative to each other to change the overlap degree thereof, and thus the width of the acting portion 311 is adjusted.

**In** an example, referring to FIG. 13A and FIG. 13B, the control system 33 may also include a communication unit 332. The communication unit 332 is configured to establish a wireless communication with a control device located either internally or externally to the patient's body through a wireless way. For example, the communication unit 332 may, but not limited to, communicate with the external control device through the following ways: Bluetooth, a cellular network, Wi-Fi, radio frequency communication, or ultrasonic communication, and the like.

A doctor (or a patient) may operate the control device to send a control instruction to the communication unit 332. The communication unit 332 is configured to receive this instruction and transmit it to the control unit 341. Upon receiving the control instruction, the control unit 341 is configured to control the adjustment mechanism to adjust the width of the acting portion 311 based on the control instruction.

After the medical device provided by the present invention is implanted, a patient may undergo periodic follow-up examinations to confirm the change of the condition. When it is detected that the width of the acting portion no longer matches with the patient's current condition, the doctor may send a control instruction via the control device located either internally or externally to the patient's body to the communication unit of the medical device implanted in the patient's body. Upon receiving this instruction, the control unit may control the adjustment mechanism according to this instruction, so that the adjustment mechanism adjusts the width of the acting portion appropriately, and thus the pad 311 matches with the patient's condition again.

In another example, referring again to FIG. 13A and FIG. 13B, the medical device may also include a sensor 35, the sensor 35 is in communication with the control unit 341. The sensor 35 is configured to sense the patient's physiological information, and send the sensed physiological information to the control unit 341 to make the control unit 341 to control the adjustment mechanism to adjust the width of the acting portion 311 based on this physiological information.

For example, this physiological information may include, but is not limited to, one or more of the following: blood pressure information, blood flow velocity information, blood pH information, blood temperature information, blood oxygen information, electrocardiographic information, heart sound information, heart acceleration information and myocardial contractility information of the heart. Correspondingly, the sensor in the present invention may be a sensor capable of sensing one or more of the above-mentioned types of information.

Since there is a sensor for sensing the physiological information that reflects the patient's condition, the control unit 331 is able to control the adjustment mechanism to adjust the width of the acting portion based on this physiological information, so that the width of the acting portion is able to always match with the patient's condition. By using the medical device provided by this implementation, the number of follow-up examinations is reduced, so that the economic burden and time cost for a patient are reduced.

It may be understood that, in some embodiments, the medical device provided by the present invention may include merely one of the communication unit 332 and the sensor 35 individually, or it may include both the communication unit and the sensor 35, which is not limited by the present invention. In some embodiments, the sensor may be a portion of the medical device provided by the present invention, or may be a portion of other medical device, which is not limited by the present invention.

The sensor may be implemented through various ways, which is not limited by the present invention. As an example, the sensor may include a hemodynamic sensor, such as a pressure sensor or an accelerometer. The sensor may be attached to the top of the coaptation member to sense physiological information that is able to reflect a blood flow state at the valve site. In other words, the physiological information sensed by the sensor is able to reflect the blood flow state at the valve site, that is, it is able to indicate whether there is regurgitation or stenosis phenomenon. In this way, the control unit is able to appropriately adjust the width of the acting portion based on this physiological information to make the width of the acting portion better match with the patient's current condition.

It should be noted that, in other examples, the sensor may not be attached to the coaptation member. For example, in some embodiments, the sensor may be mounted on the leaflet or the extension portion. For another example, in some embodiments, the sensor may also be mounted on other members of the medical device.

Additionally, it should also be noted that, in some embodiments, the medical device provided by the present invention may also include a plurality of sensors, and the plurality of sensors may be arranged at different positions to more accurately determine the patient's condition.

Considering energy consumption reduction, in an example, with reference to FIG. 13A and FIG. 13B again, the control system 33 may also include a wake-up unit 333. The wake-up unit 333 is configured to wake up one or more of the control unit 341, the communication unit 332 and the sensor 35 according to a preset schedule.

Considering that the patient's condition usually develops slowly, it is not necessary to frequently adjust the width of the acting portion. In this implementation, the control unit, the sensor and the communication unit may be in a dormant state for most of the time. When a preset time point is reached, the wake-up unit may wake up one or more of them. In this way, the energy consumption may be reduced to achieve the purpose of energy saving.

As an example, the preset schedule may include a plurality of cycles, each cycle includes a sleep phase and a wake-up phase. During the sleep phase, other energy-consuming units, except for the wake-up unit 333, may be in the dormant state to reduce energy consumption. After the wake-up phase begins, the wake-up unit 333 may wake up a portion or all of the energy-consuming units.

For example, one sleep phase may last 6 to 12 months, and one wake up phase may be 3 to 7 days. A duration and start time of each phase are adjustable. The doctor may set the preset schedule to match with the patient's follow-up plan, that is, the schedule is set to make the medical device 10 be in the wake-up phase when the patient is required to perform a follow-up examination. In this way, after the doctor has checked the patient's condition, they may send a control instruction from an external control device to the communication unit 332 to adjust the width of the acting portion 311 to match with the patient's condition after the follow-up examination. Of course, the doctor may also wake up and operate the medical device through an external device.

In an example, referring again to FIG. 13A and FIG. 13B, the control system 33 may also include an energy supply unit 334, and the energy supply unit 334 is configured to provide energy to one or more of the control unit 341, the communication unit 332, the wake-up unit 333 and the sensor 35.

The energy supply unit 334 may be implemented through various ways, which will not be limited in the present invention. For instance, in some embodiments, the energy supply unit 334 may be a battery. For another example, in some embodiments, the energy supply unit 334 may be an induction coil, the doctor or patient is able to recharge the energy supply unit 334 via an external device. For yet another example, in some embodiments, the energy supply unit 334 may be an ultrasonic transducer, which convert ultrasonic waves sent by an external device into electrical energy.

For the purpose of simplifying the structure, in some embodiments, a portion or all of the control system 33 may be located inside the acting portion 311. Certainly, in some embodiments, the acting portion 311 may be constructed to allow the doctor to manually adjust its width during the operation. A possible implementation is described below.

The method of adjusting the width of the acting portion is not limited to being achieved through the control unit 331 and adjustment mechanism. In alternative embodiments, the acting portion may be constructed such that its width may also be adjusted manually by a physician in an operation, as described in the following exemplary implementation.

Referring to FIG. 14, in this embodiment, the acting portion 411 of the coaptation member of the medical device includes a pair of shells, namely a first shell 4111 and a second shell 4112. In some embodiments, the first shell 4111 may be fixedly connected to the extension portion of the coaptation member. It should be noted that, the construction of the coaptation member and the pair of blades of the medical device provided by this embodiment may refer to the medical device 10, described in the above embodiments. For the sake of brevity, they will not be repeated herein.

The first shell 4111 is sleeved outside the second shell 4112. In this embodiment, the medical device also includes a force-applying member 43, and the force-applying member 43 is configured to apply forces on the first shell 4111 and second shell 4112 respectively to make them move toward directions away from each other. For example, the force-applying member 43 may be a compression spring 43, the compression spring 43 is provided in a cavity defined cooperatively by the first shell 4111 and second shell 4112, and two ends of the compression spring 43 respectively abut against ends of inner surfaces of the shells in their width direction.

An outer surface of the second shell 4112is provided with a pair of lugs 4112a, and an inner surface of the first shell 4111 is provided with a pair of fixing grooves. The pair of lugs 4112a are respectively match with the pair of fixing grooves (that is, the pair of lugs 4112a are inserted into the pair of fixing grooves respectively) to make the width of the acting portion 411 be adjustable.

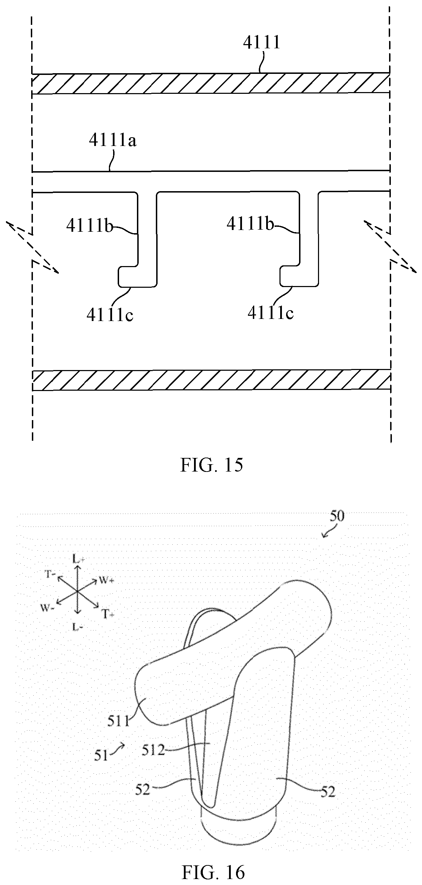

Specifically, referring to FIG. 15, each fixing groove includes a trunk 4111a extending along the width direction and a plurality of branches intersecting with the main trunk 4111a and arranged at intervals in the width direction. Each branch includes a connecting segment 4111b and a tail segment 4111c. An end of the connecting segment 4111b is connected to the main trunk 4111a, and the other of the connecting segment 4111b is connected to tail segment 4111c. An end of the tail segment 4111c is connected to the connecting segment 4111b, and the other end extends in the width direction towards a side where the shell 4112 is located.

Under a normal condition, the lug 4112a locates at the tail segment 4111c of one branch, and under the action of the force-applying member 43, the lug 4112a will not disengage from the tail segment 4111c. When it is necessary to adjust the width of the acting portion 411, the doctor may manually adjust the width (for example, by using a surgical instrument) of the acting portion 411. Specifically, the acting portion 411may firstly be compressed to move the lug 4112a from the tail segment 4111c to the connecting segment 4111b.Then, the pair of shells 4111 and 4112 may be made to rotate relative to each other to move the lug 4112a from the connecting segment 4111b to the main trunk 4111a. Finally, the pair of shells 4111 and 4112 may be made to move relatively in the width direction and rotate relatively to move the lug 4112a into the tail segment 4111c of another branch. In this way, the width of the acting portion 411 is able to be manually changed.

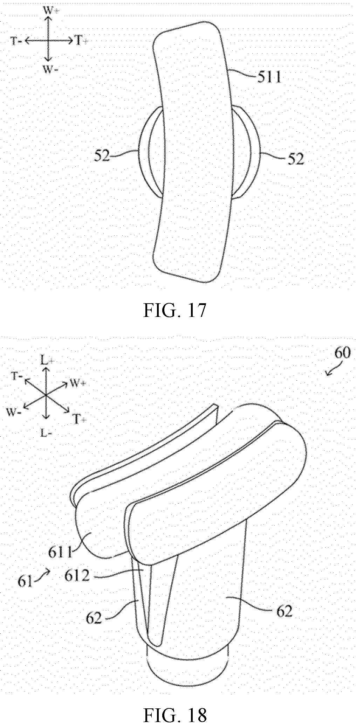

FIG. 16 and FIG. 17 show a medical device 50 according to another embodiment of the present invention. As shown in FIG. 16 and FIG. 17, the medical device 50 is substantially the same as the medical device 20 described in the previous embodiment. The medical device 50 includes a coaptation member 51 and a pair of blades 52. The pair of blades 52 is disposed on two opposite sides of the coaptation member 51 in the thickness direction. The coaptation member 51 includes an acting portion 511 and an extension portion 512.The extension portion 512 extends in the length direction, an end of the extension portion 512 is connected to the pair of blades 52, and the other end of the extension portion 512 is connected to the acting portion 511.

In this embodiment, the acting portion 511 is constructed to be bent in a manner that protrudes towards a side (that is, the T+ side) in the thickness direction. Considering that the dimensions of the pair of leaflets FL, SL are usually not exactly the same, as shown in FIG. 1B, when the pair of leaflets FL,SL coaptate, the boundary between them is typically bent in a way that protrudes towards a side, that is, roughly in a C-shape. When the medical device 50 is implanted at the patient's valve site, the pair of leaflets FL, SL are located on the two opposite sides of the acting portion 511 in the thickness direction. The acting portion 511 is constructed to be bent in a way that protrudes towards a side in the thickness direction, and this construction of the acting portion 511 helps to match with the shape of the pair of leaflets FL,SL, and thereby facilitating the proper coaptation of the pair of leaflets FL,SL. If the acting portion does not match with the shape of the pair of leaflets FL,SL, when the pair of leaflets FL,SL coaptate, the acting portion may affect the coaptation of the pair of leaflets FL,SL, so that the coaptation of the pair of leaflets FL,SL is less tight, and thus the regurgitation is caused.

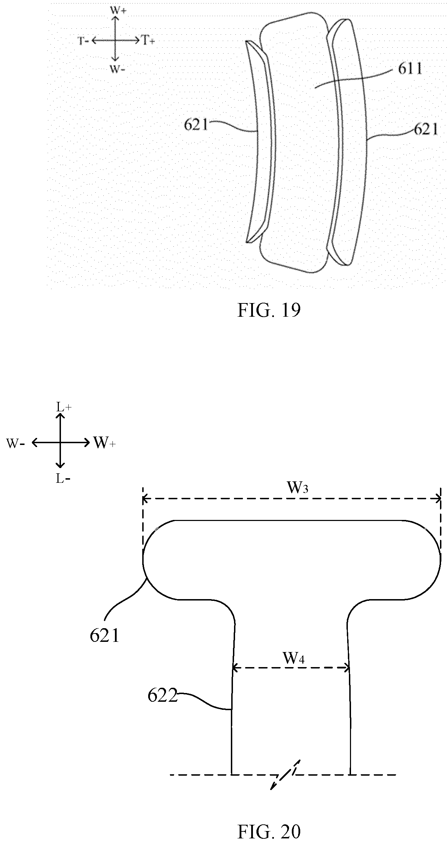

FIG. 18 and FIG. 19 show a medical device 60 according to another embodiment of the present invention. As shown in FIG. 18 and FIG. 19, the medical device 60 substantially the same as the medical device 20 described in the previous embodiment. The medical device 60 includes a coaptation member 61 and a pair of blades 62. The pair of blades 62 are disposed on two opposite sides of the coaptation member 61 in the thickness direction. The coaptation member 61 includes an acting portion 611 and an extension portion 612. The extension portion 612 extends in a length direction, and an end of the extension portion 612 in the length direction is connected to the blades 62, and the other end is connected to the acting portion 611.

In this embodiment, referring to FIG. 20, each blade 62 includes a clipping seat portion 621 and a main body portion 622. The main body portion 622 extends in the length direction. An end of the main body portion 622 in the length direction is connected to the coaptation member 61, and the other end is connected to the clipping seat portion 621. A width W₃ of the clipping seat portion 621 is greater than a width W₄ of the main body portion 622. After the medical device 60 is implanted at the patient's valve site, the clamping seat portion 621 of each blade 62 and the acting portion 611 of the coaptation member 61 and/or the main body portion of each blade 62 and the extension portion 612 of the coaptation member 61 clip a leaflet between them. Since both the clamping seat portion 621 and the acting portion 611 have a larger width, their cooperation is able to further expand the acting range of the medical device, so that the effectiveness of the medical device is improved, and thus the regurgitation is better improved.

It should be noted that, in the present invention, the width W₃ of the clipping seat portion 621 is not just slightly but rather significantly greater than the width W₄ of the main body portion 622. In this way, the medical device 60 is able to function like the multiple conventional devices in a larger heart. For example, a ratio of width W₃ to width W₄ ranges from 1.25 to 5.5. In an example, the of width W₃ to width W₄ ranges from 2 to 4. Consequently, as shown in FIG. 18 to FIG. 20, a projection of the blade 62 in the thickness direction is a T-shape.

Preferably, the clipping seat portion 621 has a structure matching with the acting portion 611. For example, when the acting portion 611 is a substantially cylindrical structure, the clipping seat portion 621 has an arcuate surface with its concave surface facing an outer surface of the acting portion 611.

Referring again to FIG. 19, the acting portion 611 of coaptation member 61 and the clipping seat portion 621 of each blade 62 are constructed to be bent in a manner that protrude toward the same side (that is, the T+ side) in the thickness direction. The medical device 60 with this structure is able to better match with the shape of the pair of leaflets FL,SL, so that the regurgitation is more effectively improved.

It should be understood that, in other embodiments, the acting portion of the coaptation member and clipping seat portion of each blade may also extend straight in the width direction without bending.

It should be understood that, the medical device according to the present invention is not limited to application at the mitral valve site. Apparently, the medical device according to the present invention may also be applied to other heart valve site. For example, the medical device according to the present invention may also be used at the tricuspid valve site. More particularly, the medical device may be applied to a pair of coaptating leaflets of the tricuspid valve.

It should be understood that, the term "include" and its variants used in the present invention are open-ended inclusions, that is, they mean "include but not limited to". The term "one embodiment" means "at least one embodiment," and the term "another embodiment" means "at least one additional embodiment."

It should be understood that, although terms such as "first" or "second" may be used in the present invention to describe various elements (for example, first chamber and second chamber), but these elements are not limited by such terms, these terms are used merely to distinguish one element from another.

It should be noted that, the various specific technical features (elements) described in the above specific embodiments may be combined in any suitable manner without contradiction. To avoid unnecessary repetition, the present invention does not separately describe the various possible combinations.

It should be understood that, multiple member s and/or portions may be provided by a single integrated member or portion. Alternatively, a single integrated member or portion may be divided into separate multiple member s and/or portions. The use of "a" or "an" to describe a member or a portion is not intended to exclude other member s or portions.

The above descriptions represent merely specific implementations of the present invention, but the scope of protection is not limited thereto. Any modifications or substitutions that can be conceived by those skilled in the art within the technical scope of the technology disclosed herein shall be covered within the protection scope of the present invention. Therefore, the protection scope of the present invention shall be subject to the scope defined by the claims.

## Claims

1. A medical device for repairing a patient' valve, comprising a coaptation member and a pair of blades disposed on two sides of the coaptation member in a thickness direction of the coaptation member, wherein the coaptation member comprises:
an extension portion that extends in a length direction, and an end of the extension portion is connected to the pair of blades; and
an acting portion that is connected to the other end of the extension portion and has a width greater than a width of the extension portion.

2. The medical device according to claim 1, wherein the width of the acting portion is adjustable.

3. The medical device according to claim 2, wherein the medical device further comprises:
a control unit; and
an adjustment mechanism configured to adjust the width of the acting portion under a control of the control unit.

4. The medical device according to claim 3, further comprising a communication unit configured to receive a control instruction, wherein the control unit is configured to control the adjustment mechanism to adjust the width of the acting portion based on the control instruction.

5. The medical device according to claim 3, further comprising a sensor configured to sense a physiological information of the patient, and the control unit is configured to control the adjustment mechanism to adjust the width of the acting portion based on the physiological information.

6. The medical device according to claim 5, wherein the control system further comprises a wake-up unit configured to wake up one or more of the control unit, the communication unit and the sensor based on a preset schedule.

7. The medical device according to claim 2, wherein the acting portion comprises a pair of shells comprising a first shell and a second shell, wherein the first shell is sleeved over an outer side of the second shell, and the adjustment mechanism is configured to adjust an overlap degree of the first shell and the second shell under the control of the control unit to adjust the width of a pad.

8. The medical device according to claim 7, wherein the adjustment mechanism comprises a driving unit, a pair of gears and a lead screw, the pair of gears comprise a first gear and a second gear meshing with each other, the driving unit is configured to drive the first gear to rotate, such that the second gear is driven to rotate, the second gear is engaged with the lead screw and is configured to drive the lead screw to rotate, and the lead screw is configured to drive the first shell and the second shell to move relatively to adjust the overlap degree of the first shell and the second shell.

9. The medical device according to claim 2, further comprising a force-applying member, wherein the acting portion comprises a pair of shells comprising a first shell and a second shell, the first shell is sleeved on an outer side of the second shell, and the force-applying member is configured to adjust an overlap degree of the first shell and the second shell to adjust the width of the pad.

10. The medical device according to claim 9, wherein an outer surface of the second shell is provided with a pair of lugs, an inner surface of the first shell is provided with a pair of fixing grooves, and the pair of fixing grooves match with the pair of lugs respectively to make the acting portion has an adjustable width.

11. The medical device according to claim 1, wherein the acting portion is bent in a manner of protruding toward a side in the thickness direction.

12. The medical device according to claim 1, wherein a projection of the coaptation member in the thickness direction is a T-shape, and the acting portion is disposed to be perpendicular to the extension portion.

13. The medical device according to any one of claims 1 to 12, wherein a ratio of the width of the acting portion to the width of the extension portion ranges from 1.25 to 5.5.

14. The medical device according to claim 1, wherein each blade comprises:
a main body portion, an end of the main body portion in the length direction being connected to the coaptation member; and
a clipping seat portion that is disposed at the other end of the main body portion, and a width of the clipping seat portion being greater than a width of the main body portion.

15. The medical device according to claim 14, wherein the acting portion and the clipping seat portion of each blade are bent in a manner of protruding toward a same side in the thickness direction.

16. The medical device according to claim 14, wherein a ratio of the width of the clipping seat portion to the width of the main body portion ranges from 1.25 to 5.5.

17. The medical device according to claim 14, wherein a projection of the blade in the thickness direction is a T-shape, and the clipping seat portion has a structure matching with the acting portion.
